# EUROPÄISCHE PATENTSCHRIFT

(11) **EP 0 659 065 B1**
(45) Veröffentlichungstag und Bekanntmachung des Hinweises auf die Patenterteilung: **15.09.1999**
(21) Anmeldenummer: 94919649.7
(22) Anmeldetag: 21.06.1994
(51) Int. Cl.: A61F 2/44

(54) **PLATZHALTER FÜR EINE BANDSCHEIBE**
SPACER FOR AN INTERVERTEBRAL DISK
ECARTEUR POUR DISQUE INTERVERTEBRAL

(30) Priorität: 09.07.1993 DE 4323034
(43) Veröffentlichungstag der Anmeldung: 28.06.1995
(73) Patentinhaber: Biedermann, Lutz, 78048 Villingen-Schwenningen (DE); Harms, Jürgen, Prof. Dr., 76337 Waldbronn (DE)
(72) Erfinder: Biedermann, Lutz, 78048 Villingen-Schwenningen (DE); Harms, Jürgen, Prof. Dr., 76337 Waldbronn (DE)
(74) Vertreter: Prüfer, Lutz H., Dipl.-Phys.
(86) Internationale Anmeldenummer: EP9402021
(87) Internationale Veröffentlichungsnummer: WO9501763

(56) Entgegenhaltungen:
- EP-A- 0 268 115
- EP-A- 0 538 183
- DE-U- 9 216 092
- SU-A- 1 424 826

## Beschreibung

Die Erfindung betrifft einen Platzhalter für eine Bandscheibe.

Aus der EP-A-0 268 115 ist ein Platzhalter für einen Wirbelkörper mit einer mantelförmigen Wandung mit einer Mehrzahl von Ausnehmungen in der Wandung und einer Mehrzahl von Zacken an den beiden Rändern der Wandung bekannt. Aus der SU-A-1 424 826 ist ferner ein Fixierelement zu Fixieren von zwei benachbarten Wirbeln relativ zueinander bekannt, welches einen keilförmigen Querschnitt aufweist.

Aufgabe der Erfindung ist es, einen Platzhalter zu schaffen, der zum Ersatz einer Bandscheibe einsetzbar ist.

Diese Aufgabe wird durch Platzhalter gemäß Patentanspruch 1 gelöst.

Weiterbildungen der Erfindung sind in den Unteransprüchen gekennzeichnet.

Weiter Merkmale und Zweckmäßigkeiten der Erfindung ergeben sich aus der Beschreibung eines Ausführungsbeispieles anhand der Figuren. Von den Figuren zeigen:
- Fig. 1: eine Seitenansicht des Platzhalters;
- Fig. 2: eine Seitenansicht des Platzhalters mit Scherlinien und getrimmter Platzhalterform;
- Fig. 3: den Platzhalter mit Scherlinien und einer abgewandelt getrimmten Form; und
- Fig. 4 bis Fig. 6: zwischen Wirbelkörper eingesetzte Platzhalter mit verschiedener Trimmung.

Der Platzhalter weist eine mantelförmige Wandung 1 auf. Der Querschnitt des von dem Mantel umgebenen Hohlraumes ist vorzugsweise oval ausgebildet. In abgewandelten Ausführungsformen ist er nierenförmig bzw. nahezu zylindrisch. Der Mantel 1 weist in der aus den Figuren ersichtlichen Weise sich mit ihrer Längsdiagonalen parallel zur Achse 2 des Platzhalters erstreckende rautenförmige Ausnehmungen 3 auf. Wie aus den Figuren ersichtlich ist, ist die Höhe der Rauten 4 an der einen Seite am größten und die Höhe der Rauten 5 an der gegenüberliegenden Seite am kleinsten. Die dazwischen liegenden Rauten sind so ausgebildet, daß die Höhe kontinuierlich von den größten Rauten zu den kleinsten Rauten abnimmt. Die an den Rändern oben und unten hervorstehenden Enden der Rauten stehen als Zacken 6 nach oben bzw. unten hervor. Wie aus den Figuren ersichtlich ist, weist jede Zacke 6 einen sich im wesentlichen parallel zur Achse 2 erstreckenden überstehenden Abschnitt 7 auf. Die Abschnitte haben im wesentlichen jeweils eine gleiche Länge. Aufgrund der unterschiedlichen Höhe der Rauten ergibt sich somit ein Platzhalter, dessen die beiden zackenförmigen Bereiche begrenzenden Ränder zueinander unter einem Winkel geneigt sind, so daß der Querschnitt des Platzhalters keilförmig ausgebildet ist. Vorzugsweise liegt der Keilwinkel im Bereich von 8° bis 120 und insbesondere im Bereich von 9° bis 11° und ganz besonders im Bereich von 10°.

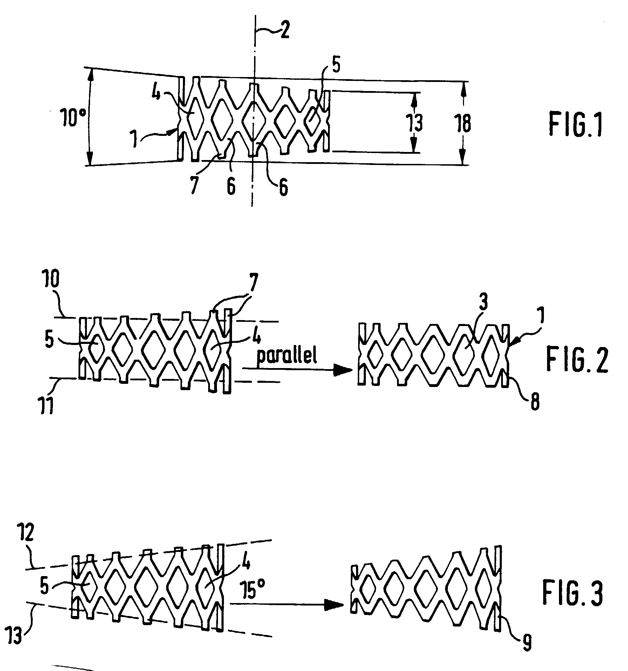

Die Länge der sich in axialer Richtung erstreckenden Abschnitte 7 ist so gewählt, daß in der aus Fig. 2 ersichtlichen Weise beim Abscheren der Abschnitte 7 entlang der Linien 10 und 11 im Bereich der Rauten 4 mit der größten Höhe und teilweisem Abschneiden der Abschnitte der Rauten, die zwischen der Raute 4 mit der größten Höhe und der Raute 5 mit der kleinsten Höhe liegen, ein Platzhalter 8 mit parallelen Rändern und somit einem rechteckigen Querschnitt entsteht. Bei dieser Länge der Abschnitte 7 kann andererseits beim Abscheren der zu den Rauten 5 mit der kleinsten Höhe und einem teilweisen Abscheren der Abschnitte der zwischen der Raute 4 mit der größten Höhe und der Raute 5 mit der kleinsten Höhe liegenden Rauten entlang der Linien 12 und 13 ein Platzhalter 9 gebildet werden, dessen Keilwinkel größer als der des Ausgangsmodelles ist und vorzugsweise im Bereich von 14° bis 16° und insbesondere bei etwa 15° liegt.

Als Material für die Platzhalter ist insbesondere ein Titanblech bzw. ein Titanrohr oder auch ein körperresorbierendes Kunststoffmaterial gewählt.

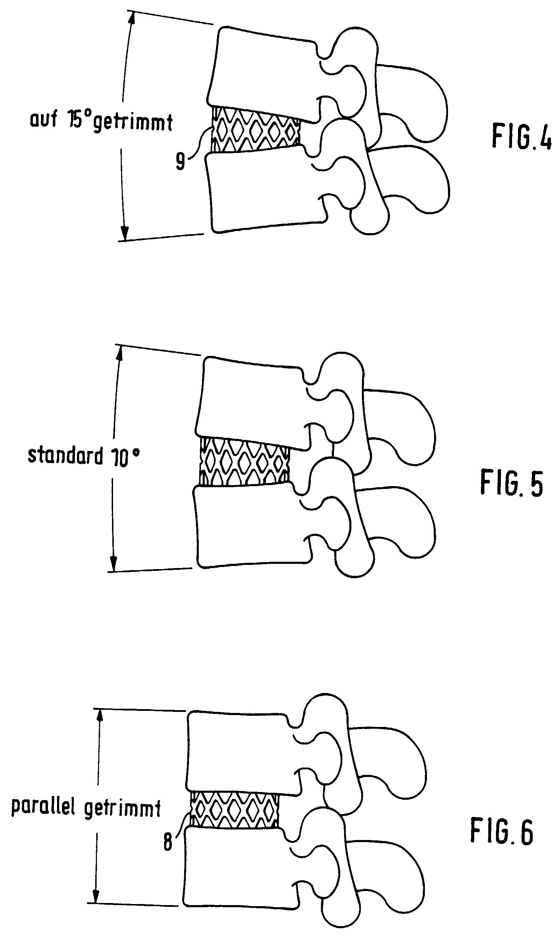

Der so beschriebene Platzhalter wird von dem Operateur durch Abschneiden der Zacken entlang der gestrichelt angedeuteten Schnittlinien 10, 11 bzw. 12, 13 zum Trimmen in die gewünschte Form 8 bzw. 9 abgeschnitten. Anschließend kann der so getrimmte Platzhalter in den Fig. 4 und 6 oder in seiner Ausgangsform in Fig. 5 zwischen die Wirbelkörper eingesetzt werden.

Die oben beschriebene Ausgestaltung des Platzhalters ermöglicht nicht nur ein leichtes Anpassen an verschiedene Keilformen eines insbesondere für die Bandscheibe zu verwendenden Platzhalters, sondern sie ermöglicht auch eine erhebliche Vereinfachung der Lagerhaltung, da der Operateur nur eine einzige Form eines Platzhalters vorrätig halten muß.

## Patentansprüche

1. Platzhalter für eine Bandscheibe, mit einer mantelförmigen Wandung (1) mit einer Mehrzahl von Ausnehmungen (4, 5) in der Wandung und einer Mehrzahl von abtrennbare Abschnitte aufweisenden Zacken (6) an den beiden Rändern der Wandung (1), wobei die beiden Ränder so gegeneinander geneigt sind, daß der Platzhalter einen keilförmigen Querschnitt aufweist und die Höhe der Ausnehmungen (4, 5) in Richtung zwischen den beiden Rändern gesehen an der einen Mantelseite von einem größten Wert zu einem an der gegenüberliegenden Mantelseite liegenden kleinsten Wert abnimmt.

2. Platzhalter, für eine Bandscheibe, nach Anspruch 1,
dadurch gekennzeichnet, daß die Ausnehmungen in der Wandung (1) als sich im wesentlichen in Axialrichtung des Hohlkörpers erstreckende Vierecke bzw. Rauten (5) ausgebildet sind.

3. Platzhalter, für eine Bandscheibe, nach Anspruch 1 oder 2,
dadurch gekennzeichnet, daß die Neigung zwischen 8° und 12° beträgt.

4. Platzhalter, für eine Bandscheibe, nach Anspruch 3,
dadurch gekennzeichnet, daß die Neigung 9° bis 11° beträgt.

5. Platzhalter, für eine Bandscheibe, nach einem der Ansprüche 1 bis 4,
dadurch gekennzeichnet, daß der Querschnitt des von dem Mantel umgebenen Holraumes oval ausgebildet ist.

## Claims

1. Spacer for intervertebral disc, with a shell-like wall (1) with a plurality of cut-outs (4, 5) in the wall and a plurality of teeth (6) with portions which can be taken off on the two edges of the wall (1), wherein the two edges are inclined relative to one another so that the spacer has a wedge-shaped cross-section and the height of the cut-outs (4, 5) viewed in the direction between the two edges on one side of the shell decreases from a greatest value to a smallest value on the opposite side of the shell.

2. Spacer for an intervertebral disc according to Claim 1, characterised in that the cut-outs in the wall (1) are constructed as quadrilaterals or rhombi (5) which extend substantially in the axial direction of the hollow body.

3. Spacer for an intervertebral disc according to Claim 1 or 2, characterised in that the inclination is between 8° and 12°.

4. Spacer for an intervertebral disc according to Claim 3, characterised in that the inclination is between 9° and 11°.

5. Spacer for an intervertebral disc according to one of Claims 1 to 4, characterised in that the cross-section of the cavity surrounded by the shell is oval.

## Revendications

1. Dispositif de positionnement pour un disque intervertébral, comprenant une paroi (1) à surface convexe avec une pluralité d'évidements (4, 5) réalisés dans la paroi et une pluralité de dents (6), réalisées sur les deux bords de la paroi (1) et munies de parties qui peuvent être tronquées, dans lequel les deux bords sont inclinés l'un vers l'autre de façon telle que le dispositif de positionnement présente une section de forme conique et la hauteur des évidements (4, 5), considérée dans la direction entre les deux bords, diminue de la plus grande valeur sur un des côtés de la paroi jusqu'à la plus petite valeur sur le côté opposé de la paroi.

2. Dispositif de positionnement pour un disque intervertébral selon la revendication 1, caractérisé en ce que les évidements réalisés dans la paroi (1) sont conçus sous forme de rectangles ou losanges (5) qui s'allongent sensiblement dans le sens axial du corps creux.

3. Dispositif de positionnement pour un disque intervertébral selon la revendication 1 ou 2, caractérisé en ce que l'inclinaison est comprise entre 8° et 12°.

4. Dispositif de positionnement pour un disque intervertébral selon la revendication 3, caractérisé en ce que l'inclinaison est comprise entre 9° et 11°.

5. Dispositif de positionnement pour un disque intervertébral selon l'une quelconque des revendications 1 à 4, caractérisé en ce que le volume intérieur délimité par la paroi présente une section ovale.
